(19) Europäisches Patentamt
European Patent Office
Office européen des brevets

(11) **EP 2 700 663 A1**

(12) **EUROPEAN PATENT APPLICATION**
published in accordance with Art. 153(4) EPC

(43) Date of publication:
**26.02.2014 Bulletin 2014/09**

(21) Application number: **12774301.1**

(22) Date of filing: **19.04.2012**

(51) Int Cl.:
*C08F 20/06* (2006.01)        *A61F 13/49* (2006.01)
*A61F 13/53* (2006.01)        *B01J 20/26* (2006.01)
*B32B 27/00* (2006.01)

(86) International application number:
**PCT/JP2012/060627**

(87) International publication number:
**WO 2012/144564 (26.10.2012 Gazette 2012/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.04.2011 JP 2011095299**

(71) Applicant: **Sumitomo Seika Chemicals Co., Ltd.
Kako-gun Hyogo 675-0145 (JP)**

(72) Inventors:
• **KONDO, Kimihiko
Himeji-shi, Hyogo 672-8076 (JP)**
• **UMEZA, Ryusuke
Himeji-shi, Hyogo 672-8076 (JP)**
• **TAKAMATSU, Kazuyoshi
Himeji-shi, Hyogo 672-8076 (JP)**
• **TAKATORI, Junichi
Himeji-shi, Hyogo 672-8076 (JP)**

(74) Representative: **Vossius & Partner
Siebertstrasse 4
81675 München (DE)**

(54) **WATER-ABSORBENT RESIN, ABSORBENT BODY AND ABSORBENT ARTICLE**

(57) The present invention provides a novel water-absorbent resin desirable for use as a hygienic material and the like, and an absorbent material and absorbent article using the water-absorbent resin; the water-absorbent resin having a high gel strength that prevents a reduction in liquid permeation rate by suppressing gel blocking during absorption, and an excellent water-retention capacity at the same time.

The water-absorbent resin satisfies the following criteria (1) and (2):

(1) having a water-retention capacity of saline solution of 40 g/g or more, and

(2) having a total gel strength of 5,500 Pa or greater, the total gel strength being the sum of 30 times swollen gel strength **a,** 40 times swollen gel strength **b,** and 50 times swollen gel strength **c.**

EP 2 700 663 A1

**Description**

Technical Field

The present invention relates to a water-absorbent resin, and to an absorbent material and absorbent article using the water-absorbent resin. More specifically, the present invention relates to a water-absorbent resin that has a high water-retention capacity and a high gel strength, and that is desirable for use in a hygienic material and the like, and the present invention further relates to an absorbent material and absorbent article using the water-absorbent resin.

Background Art

In recent years, water-absorbent resin has been widely used in various fields, including hygienic materials, such as disposable diapers and sanitary napkins, agricultural and horticultural materials, such as water-retaining agents and soil conditioners, and industrial materials, such as water-blocking agents and agents for preventing dew condensation. Of these, water-absorbent resin is frequently used in, in particular, hygienic materials, such as disposable diapers and sanitary napkins.

As the water-absorbent resin, for example, hydrolysates of starch-acrylonitrile graft copolymers, neutralized products of starch-acrylate graft polymers, saponified products of vinyl acetate-acrylic ester copolymers, and crosslinked products of partially neutralized acrylic acid polymers are known.

In recent years, the absorbent materials in hygienic articles, such as disposable diapers and sanitary napkins, have tended to become thinner to achieve a more comfortable feeling in use. Examples of methods for thinning an absorbent material include a method for increasing the proportion of water-absorbent resin in an absorbent material, a method for improving the water-retention capacity of a water-absorbent resin, and the like.

In a method for increasing the proportion of water-absorbent resin in an absorbent material, the following disadvantage exists when liquid distribution or diffusion that occurs in the actual use of the absorbent material is considered. That is, when a large amount of the water-absorbent resin turns into a soft gel by the absorption of a liquid, the water-absorbent resin near the surface layer absorbs the liquid, and the soft gel around the surface layer is further densified. This inhibits the permeation of the liquid into the interior of the absorbent material, preventing the internal water-absorbent resin from efficiently absorbing the liquid. This phenomenon, which is called "gel blocking," markedly lowers liquid diffusibility and slows the liquid permeation rate of the absorbent material. Therefore, a water-absorbent resin is required to have a high gel strength to suppress the occurrence of gel blocking. However, to increase the gel strength of a water-absorbent resin, it is generally required to increase the crosslinking density of the water-absorbent resin, which results in a decreased water-retention capacity for the water-absorbent resin.

Meanwhile, a method for lowering the crosslinking density of a water-absorbent resin is considered as a method for improving the water-retention capacity of the water-absorbent resin. However, this method results in a decrease in the gel strength of the water-absorbent resin. If such a water-absorbent resin is used in an absorbent material, the leakage of body fluid occurs as a result of the insufficient gel strength of the water-absorbent resin, causing a loss of comfort to the wearer.

In order to solve these problems of absorbent materials, some production methods have been proposed for the purpose of improving the properties of water-absorbent resin. For example, known methods include a method for performing reversed-phase suspension polymerization using a specific amount of a specific surfactant (see Patent Literature 1); a method for performing reversed-phase suspension polymerization in two or more steps (see Patent Literature 2); a method for performing reversed-phase suspension polymerization in the presence of ß-1,3-glucan to obtain a water-absorbent resin, and adding a crosslinking agent to the obtained water-absorbent resin to cause a crosslinking reaction (see Patent Literature 3); a method for performing reversed-phase suspension polymerization using a specific amount of a persulfate as a polymerization initiator (see Patent Literature 4); and a method for performing an aqueous solution polymerization in the presence of phosphorous acid and/or a salt thereof to obtain a water-absorbent resin precursor, and then mixing and heating the water-absorbent resin precursor with a surface-crosslinking agent (see Patent Literature 5).

However, the water-absorbent resins produced by these methods have a low gel strength when a high water-retention capacity is attained, and are thus insufficient to achieve the required properties as an absorbent material.

Citation List

Patent Literature

PTL 1: Japanese Unexamined Patent Publication No. H6-345819
PTL 2: Japanese Unexamined Patent Publication No. H3-227301
PTL 3: Japanese Unexamined Patent Publication No. H8-120013
PTL 4: Japanese Unexamined Patent Publication No. H6-287233
PTL 5: Japanese Unexamined Patent Publication No. H9-124710

Summary of Invention

Technical Problem

The present invention has been made in view of the current state of the aforementioned prior art. A principal object of the present invention is to provide a novel water-absorbent resin desirable for use as a hygienic material and the like, and an absorbent material and absorbent article using the water-absorbent resin; the water-absorbent resin having excellent water-retention capacity, as well as a high gel strength capable of preventing a reduction in liquid permeation rate by suppressing gel blocking during absorption.

Solution to Problem

The present inventors conducted extensive research to achieve the above object. As a result, the inventors found that optimizing the production conditions in the polymerization of a water-soluble ethylenic unsaturated monomer to produce a water-absorbent resin can yield an unconventional novel water-absorbent resin having a high water-retention capacity as well as a high gel strength. The present invention has thereby been accomplished.

Specifically, the present invention provides the water-absorbent resin, and the absorbent material and absorbent article using the water-absorbent resin described below.

Item 1. A water-absorbent resin satisfying criteria (1) and (2) below:

(1) having a water-retention capacity of saline solution of 40 g/g or more, and
(2) having a total gel strength of 5,500 Pa or greater, the total gel strength being the sum of 30 times swollen gel strength **a,** 40 times swollen gel strength **b,** and 50 times swollen gel strength **c,**
the 30 times swollen gel strength **a** being a gel strength when swelling being performed by adding saline solution to a gel in an amount with the total mass of the gel and saline solution being 30 times that of the gel,
the 40 times swollen gel strength b being a gel strength when swelling being performed by adding saline solution to a gel in an amount with the total mass of the gel and saline solution being 40 times that of the gel, and
the 50 times swollen gel strength c being a gel strength when swelling being performed by adding saline solution to a gel in an amount with the total mass of the gel and saline solution being 50 times that of the gel.

Item 2. An absorbent material comprising the water-absorbent resin of Item 1 and hydrophilic fiber.
Item 3. An absorbent article comprising the absorbent material of Item 2 held between a liquid-permeable sheet and a liquid-impermeable sheet.

The following describes, in detail, the water-absorbent resin of the present invention and the method for producing the water-absorbent resin.

<u>Water-Absorbent Resin</u>

The water-absorbent resin of the present invention has a high water-retention capacity and, in particular, has a water-retention capacity of saline solution of 40 g/g or more, preferably 40 to 60 g/g, and more preferably 43 to 55 g/g. If an absorbent material or absorbent article is produced using a water-absorbent resin having a water-retention capacity of saline solution of lower than the above-mentioned range, the produced absorbent material will have a low absorption capacity. This causes a failure in the absorption of a large amount of liquid, resulting in liquid leakage, re-wetting, or the like, which tends to deteriorate the comfort of the wearer of the absorbent article.

The water-retention capacity of saline solution of the water-absorbent resin is a value measured according to the measurement method described later in "Water-Retention Capacity of Saline Solution."

The water-absorbent resin of the present invention has a total gel strength of 5,500 Pa or greater, preferably 5,500 to 10,000 Pa, and more preferably 6,000 to 9,000 Pa. The total gel strength is a value expressed as the sum of **a** representing 30 times swollen gel strength, **b** representing 40 times swollen gel strength, and c representing 50 times swollen gel strength, as measured according to the measurement method described later in the "Gel Strength of Water-Absorbent Resin." In short, the total gel strength is obtained by the formula: Total gel strength (Pa) **= a + b + c.** As used herein, the 30 times swollen gel strength **a** represents a gel strength when swelling being performed by adding saline solution to a gel in an amount with the total mass of the gel and saline solution being 30 times that of the gel, the 40 times swollen gel strength **b** represents a gel strength when swelling being performed by adding saline solution to a gel in an amount with the total mass of the gel and saline solution being 40 times that of the gel, and the 50 times swollen gel strength c represents a gel strength when swelling being performed by adding saline solution to a gel in an amount with the total mass of the gel and saline solution being 50 times that of the gel.

As for the properties of the absorbent material under a load, liquid permeation rate is important in addition to the properties thereof in relation to re-wetting or liquid leakage. One of the factors that controls the liquid permeation rate is the gel blocking of the water-absorbent resin that occurs in the absorbent material. The gel blocking phenomenon is related to gel strength; the higher the gel strength, the lower the degree of gel blocking, thus achieving a satisfactory liquid permeation rate. Therefore, a resin having a high gel strength is preferable.

Generally, the gel strength is in a trade-off relationship with the swelling ratio of the water-absorbent resin. That is, an increase in the swelling ratio decreases the gel strength. A swelling ratio of 30 to 50 times is considered to have an effect on the liquid permeation rate in the absorbent material. Specifically, when the swelling ratio is less than 30 times, the amount of absorption is small, and thus, gel blocking does not occur. When the swelling ratio exceeds 50 times, gel blocking is likely to occur, which is not practical for use as a general absorbent material. Therefore, the properties of the absorbent material can be determined by calculating the total gel strength of swollen gel within the range of 30 to 50 times swelling resulting from liquid absorption in the absorbent material. As used herein, the swelling ratio refers to the ratio of the total mass of the water-absorbent resin and the absorbed liquid, relative to the mass of the water-absorbent resin.

When the thus-obtained total gel strength of the water-absorbent resin is less than 5,500 Pa, the gel strength is insufficient. Therefore, in the absorbent material or the absorbent article, during liquid absorption under a load, permeation of the liquid is inhibited due to gel blocking, resulting in an unsatisfactory liquid permeation rate.

The water-absorbent resin of the present invention has a median particle size of preferably 200 to 600 $\mu$m, more preferably 250 to 550 $\mu$m, and still more preferably 300 to 500 $\mu$m. The median particle size of the water-absorbent resin is a value measured according to the measurement method described later in "Median Particle Size."

Method for Producing Water-Absorbent Resin

The method for producing the water-absorbent resin of the present invention is not particularly limited, and the water-absorbent resin of the present invention may be produced by, for example, a reversed-phase suspension polymerization method, an aqueous solution polymerization method, or the like.

The following describes, in detail, a method for producing the water-absorbent resin of the present invention by a reversed-phase suspension polymerization method.

In a reversed-phase suspension polymerization method, a water-soluble ethylenic unsaturated monomer is mixed and heated with a radical polymerization initiator and, if necessary, a crosslinking agent and a chain transfer agent, in a petroleum hydrocarbon dispersion medium in the presence of a dispersion stabilizer, to cause polymerization.

(1) Starting Compound

(i) Water-Soluble Ethylenic Unsaturated Monomer

Examples of the water-soluble ethylenic unsaturated monomer used as a starting material include (meth)acrylic acid (as used herein, "acryl" and "methacryl" collectively refer to "(meth)acryl;" the same applies hereinafter) and salts thereof; 2-(meth)acrylamide-2-methylpropanesulfonic acid and salts thereof; (meth)acrylamide, N,N-dimethyl (meth) acrylamide, 2-hydroxyethyl(meth)acrylate, N-methylol(meth)acrylamide, polyethylene glycol mono(meth)acrylate, and like nonionic monomers; N,N-diethylaminoethyl(meth)acrylate, N,N-diethylaminopropyl(meth)acrylate, diethylaminopropyl(meth)acrylamide, and like amino group-containing unsaturated monomers, and quaternary compounds thereof. As the water-soluble ethylenic unsaturated monomer, at least one compound selected from these compounds can be used.

In particular, (meth)acrylic acid, salts thereof, (meth)acrylamide, N,N-dimethylacrylamide, etc., are preferable, and (meth)acrylic acid, salts thereof, acrylamide, etc., are more preferable.

The water-soluble ethylenic unsaturated monomer can be used in an aqueous solution to increase the dispersion efficiency in a petroleum hydrocarbon dispersion medium when reversed-phase suspension polymerization is carried out. The concentration of the monomer in an aqueous solution is not particularly limited, and usually 20% by mass or more to a saturated concentration or less, preferably 25 to 70% by mass, and more preferably 30 to 55% by mass.

When the water-soluble ethylenic unsaturated monomer contains an acid group as in (meth)acrylic acid, 2-(meth) acrylamide-2-methylpropanesulfonic acid, or the like, the acid group may be neutralized in advance with an alkaline neutralizing agent. Examples of the alkaline neutralizing agent include an aqueous solution of sodium hydroxide, potassium hydroxide, ammonia, or the like. These alkaline neutralizing agents may be used singly, or in a combination of two or more thereof.

The degree of neutralization of all the acid groups of the water-soluble ethylenic unsaturated monomer with the alkaline neutralizing agent is preferably 10 to 100% by mol, and more preferably 30 to 80% by mol, in order to increase the osmotic pressure of the resulting water-absorbent resin and to thereby improve the absorption property thereof, and also in order not to cause any disadvantages in safety or the like due to the presence of an excess alkaline neutralizing agent.

(ii) Petroleum Hydrocarbon Dispersion Medium

Examples of usable petroleum hydrocarbon dispersion media include n-hexane, n-heptane, 2-methylhexane, 3-methylhexane, 2,3-dimethylpentane, 3-ethylpentane, n-octane, and like aliphatic hydrocarbons; cyclohexane, methylcyclohexane, cyclopentane, methylcyclopentane, trans-1,2-dimethylcyclopentane, cis-1,3-dimethylcyclopentane, trans-1,3-dimethylcyclopentane, and like alicyclic hydrocarbons; and benzene, toluene, xylene, and like aromatic hydrocarbons. These petroleum hydrocarbon dispersion media may be used singly, or in a combination of two or more thereof. Examples of a mixed hydrocarbon dispersion medium comprising a mixture of two or more petroleum hydrocarbon dispersion media include Exxsol Heptane (produced by Exxon Mobil Corporation, a mixed hydrocarbon dispersion medium containing, as main components, n-heptane, 2-methylhexane, 3-methylhexane, and methylcyclohexane). Of these petroleum hydrocarbon dispersion media, n-hexane, n-heptane, cyclohexane, Exxsol Heptane, and the like, are preferably used because they are easily available industrially, stable in quality, and inexpensive.

The petroleum hydrocarbon dispersion medium is used in an amount of preferably 50 to 600 parts by mass, and more preferably 80 to 550 parts by mass, relative to 100 parts by mass of the water-soluble ethylenic unsaturated monomer, to remove the heat of polymerization and to easily control the polymerization temperature.

(iii) Dispersion Stabilizer

As a dispersion stabilizer, a surfactant can be used. Examples of surfactants include sorbitan fatty acid esters, polyglyceryl fatty acid esters, sucrose fatty acid esters, sorbitol fatty acid esters, polyoxyethylene alkyl phenyl ethers, and like nonionic surfactants; fatty acid salts, alkylbenzene sulfonates, alkyl methyl taurates, polyoxyethylene alkyl phenyl ether sulfates, polyoxyethylene alkyl ether sulfonates, and like anionic surfactants; and the like.

Of the above-mentioned surfactants, sorbitan fatty acid esters, polyglyceryl fatty acid esters, and sucrose fatty acid esters are preferable, in terms of the dispersion stability of the monomer. These surfactants may be used singly, or in a combination of two or more thereof.

The surfactant is used in an amount of 0.1 to 5 parts by mass, preferably 0.2 to 3 parts by mass, relative to 100 parts by mass of the water-soluble ethylenic unsaturated monomer, so that in the petroleum hydrocarbon dispersion medium, an excellent dispersion state of the water-soluble ethylenic unsaturated monomer is maintained, and a dispersion effect that corresponds to the amount used is obtained.

Together with a surfactant, a polymeric dispersion agent can be used as a dispersion stabilizer. Examples of the polymeric dispersion agents include ethyl cellulose, ethyl hydroxyethyl cellulose, polyethylene oxide, maleic anhydride-modified polyethylene, maleic anhydride-modified ethylene-propylene copolymer, maleic anhydride-modified polybutadiene, maleic anhydride-modified EPDM (ethylene-propylene-diene-methylene copolymer), and the like.

The polymeric dispersion agent is used in an amount of preferably 0.1 to 5 parts by mass, more preferably 0.2 to 3 parts by mass, relative to 100 parts by mass of the water-soluble ethylenic unsaturated monomer, so that in the petroleum hydrocarbon dispersion medium, an excellent dispersion state of the monomer is maintained, and a dispersion effect that corresponds to the amount used is obtained.

(iv) Radical Polymerization Initiator

Examples of radical polymerization initiators include persulfates, such as potassium persulfate, ammonium persulfate, and sodium persulfate; peroxides, such as methyl ethyl ketone peroxide, methyl isobutyl ketone peroxide, di-t-butyl peroxide, t-butyl cumyl peroxide, t-butyl peroxyacetate, t-butyl peroxyisobutylate, t-butyl peroxypivalate, and hydrogen peroxide; and azo compounds, such as 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazoline-2-yl]propane}dihydrochloride, 2,2'-azobis[N-(2-carboxyethyl)-2-methylpropionamidine]tetrahydrate, 2,2'-azobis{2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid).

Of these radical polymerization initiators, 2,2'-azobis(2-amidinopropane)dihydrochloride, 2,2'-azobis{2-[1-(2-hydroxyethyl)-2-imidazoline-2-yl]propane}dihydrochloride, and 2,2'-azobis[N-(2-carboxyethyl)-2-methylpropionamidine] tetrahydrate are preferable from the viewpoint of obtaining a water-absorbent resin having a high water-retention capacity and a high gel strength. These radical polymerization initiators may be used singly, or in a combination of two or more thereof.

Usually, the amount of the radical polymerization initiator used is preferably 0.00005 to 0.0002 mol per mole of water-soluble ethylenic unsaturated monomer, with 0.00006 to 0.00019 mol being more preferable. It is not preferable when the amount used is too small, because the polymerization reaction will require a long period of time. It is not preferable when the amount used is too large, because an abrupt polymerization reaction will occur.

The radical polymerization initiator can also be used as a redox polymerization initiator together with a reducing agent, such as sodium sulfite, sodium hydrogen sulfite, ferrous sulfate, and L-ascorbic acid.

(v) Crosslinking Agent

In the present invention, a polymerization reaction is preferably performed in the presence of a crosslinking agent (hereinafter sometimes referred to as an "internal-crosslinking agent"). Examples of internal-crosslinking agents include unsaturated polyesters obtained by reacting a polyol, for example, diol or triol, such as, (poly)ethylene glycol ("(poly)" refers to both cases with and without a prefix "poly;" hereafter the same), (poly)propylene glycol, 1,4-butanediol, trimethylolpropane, or (poly)glycerol, with an unsaturated acid, such as (meth)acrylic acid, maleic acid, or fumaric acid; bisacrylamides, such as N,N'-methylenebisacrylamide; di- or tri(meth)acrylic esters obtained by reacting a polyepoxide with a (meth)acrylic acid; carbamyl esters of di(meth)acrylic acids obtained by reacting a polyisocyanate, such as tolylenediisocyanate or hexamethylenediisocyanate, with hydroxyethyl(meth)acrylate; compounds having two or more polymerizable unsaturated groups, such as allylated starch, allylated cellulose, diallyl phthalate, N,N',N"-triallyl isocyanurate, and divinylbenzene; diglycidyl ether compounds, such as (poly)ethylene glycol diglycidyl ethers, (poly)propylene glycol diglycidyl ethers, and (poly)glycerol diglycidyl ethers; epihalohydrin compounds such as epichlorohydrin, epibromohydrin, and $\alpha$-methylepichlorohydrin; compounds having two or more reactive functional groups, for example, isocyanate compounds, such as 2,4-tolylenediisocyanate and hexamethylenediisocyanate; oxetane compounds, such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol; and the like. These internal-crosslinking agents may be used singly, or in a combination of two or more thereof.

The amount of the internal-crosslinking agent used is preferably 0.000001 to 0.001 mol per mole of water-soluble ethylenic unsaturated monomer from the viewpoint of sufficiently improving the water-retention capacity and the gel strength of the resulting water-absorbent resin.

(vi) Chain Transfer Agent

In the present invention, it is preferable to perform a polymerization reaction in the presence of a chain transfer agent. The type of chain transfer agent is not particularly limited. Examples thereof include thiols, such as ethanethiol, propanethiol, and dodecanethiol; thiolic acids, such as thioglycolic acid, thiomalic acid, dimethyldithiocarbamic acid, diethyldithiocarbamic acid, and salts thereof; secondary alcohols, such as isopropanol; phosphorous compounds; and the like.

Of these chain transfer agents, examples of phosphorous compounds include phosphorous acid compounds, such as normal salts of phosphorous acid (for example, as disodium phosphite, dipotassium phosphite, diammonium phosphite, etc.), acidic salts of phosphorous acid (for example, sodium hydrogen phosphite, potassium hydrogen phosphite, ammonium hydrogen phosphite, etc.), and phosphorous acid; phosphoric acid compounds, such as normal salts of phosphoric acid (for example, sodium phosphate, potassium phosphate, ammonium phosphate, etc.), acidic salts of phosphoric acid (for example, sodium dihydrogen phosphate, potassium dihydrogen phosphate, ammonium dihydrogen phosphate, disodium hydrogen phosphate, dipotassium hydrogen phosphate, diammonium hydrogen phosphate, etc.), and phosphoric acid; hypophosphorous acid compounds, such as hypophosphorous acid salts (for example, sodium hypophosphite, potassium hypophosphite, ammonium hypophosphite, etc.) and hypophosphorous acid; pyrophosphoric acid, tripolyphosphoric acid, polyphosphoric acid, and salts thereof; trimethyl phosphate, nitrilotris(methylenetriphosphonic acid); and the like. Further, hydrates of the phosphorous compounds may also be used.

These chain transfer agents may be used singly, or in a combination of two or more thereof.

Among chain transfer agents, thiomalic acid, isopropanol, phosphorous acid compounds, phosphoric acid compounds, and hypophosphorous acid compounds are preferable and disodium phosphite, sodium dihydrogen phosphate, and sodium hypophosphite are particularly preferable because the addition thereof improves the effect that is achieved and results in the obtainment of a water-absorbent resin having a high water-retention capacity and a high gel strength.

The amount of the chain transfer agent used is preferably 0.00001 to 0.0002 mol per mole of water-soluble ethylenic unsaturated monomer used, with 0.00002 to 0.00019 mol being more preferable. When the amount of the chain transfer agent used is too small, a water-absorbent resin having a high water-retention capacity and a high gel strength tends to be unobtainable. Further, when the amount used is too large, an effect that reflects the amount added tends to be unachievable.

(2) Reversed-Phase Suspension Polymerization Method

In the present invention, a water-soluble ethylenic unsaturated monomer may be polymerized by the reversed-phase suspension polymerization method using the above-described radical polymerization initiator in a petroleum hydrocarbon dispersion medium containing the above-described dispersion stabilizer. At this time, in order to obtain a water-absorbent resin having a high gel strength and an excellent water-retention capacity, it is preferable to perform polymerization reaction in the presence of an internal-crosslinking agent, a chain transfer agent, and the like under the above-described conditions.

In the present invention, water in an amount of 10 to 200 parts by mass may be used relative to 100 parts by mass of the above-described petroleum hydrocarbon dispersion medium. The amount of water used is preferably 10 parts by mass or more from the viewpoint of improving the dispersion state of the water-soluble ethylenic unsaturated monomer. Also, the amount of water used is preferably 200 parts by mass or less from the viewpoint of improving industrial production, which is economically desirable.

The reaction temperature of the polymerization reaction varies depending on the radical polymerization initiator used. It is not preferable when the reaction temperature is too low (below 20°C), because the polymerization time will be long. The upper limit of the reaction temperature is preferably 110°C in order to remove the heat of polymerization and perform a smooth polymerization reaction. From these viewpoints, the reaction temperature is usually 20 to 110°C, and preferably 40 to 80°C. Usually, the reaction time is preferably 0.5 to 4 hours.

In the present invention, the reversed-phase suspension polymerization may be performed in one step or in multiple steps of two or more. The number of steps is preferably 2 or 3 from the viewpoint of improving productivity.

Reversed-phase suspension polymerization in each of the second and subsequent steps may be performed by the same method as in the first step by mixing a water-soluble ethylenic unsaturated monomer with the reaction mixture obtained by the polymerization reaction in the first step after reversed-phase suspension polymerization in the first step is performed by the above-described method. The reversed-phase suspension polymerization in each of the second and subsequent steps may be performed under the same conditions as described in the above method by adding a radical polymerization initiator, an internal-crosslinking agent, a chain transfer agent, and the like, in addition to the water-soluble ethylenic unsaturated monomer, in the above-described range of molar ratio of each component relative to the water-soluble ethylenic unsaturated monomer, based on the amount of the water-soluble ethylenic unsaturated monomer added during reversed-phase suspension polymerization in each of the second and subsequent steps.

(3) Post-Crosslinking

In the present invention, in order to obtain a desired water-absorbent resin having a high gel strength and an excellent water-retention capacity, it is preferable to add a post-crosslinking agent and carry out a post-crosslinking treatment in the process after the completion of polymerization of the water-soluble ethylenic unsaturated monomer and before drying of the resulting resin.

Any post-crosslinking agent may be used as long as it can react with a carboxyl group in the water-absorbent resin. Typical examples of post-crosslinking agents include polyols, such as (poly)ethylene glycol, (poly)propylene glycol, 1,4-butanediol, trimethylolpropane, and (poly)glycerol; diglycidyl ether compounds, such as (poly)ethylene glycol diglycidyl ethers, (poly)propylene glycol diglycidyl ethers, and (poly)glycerol diglycidyl ethers; epihalohydrin compounds, such as epichlorohydrin, epibromohydrin, and α-methylepichlorohydrin; and compounds having two or more reactive functional groups, for example, isocyanate compounds, such as 2,4-tolylenediisocyanate and hexamethylenediisocyanate. These can be used singly, or in a combination of two or more thereof. Further, the post-crosslinking agent may be dissolved in water, an organic solvent, or the like for use.

The amount of the post-crosslinking agent cannot be generally specified because it varies depending on the type of post-crosslinking agent. However, when the amount of the post-crosslinking agent is too small, the surface crosslinking density of the water-absorbent resin is insufficient, weakening the gel strength. When the amount of the post-crosslinking agent used is too large, the water-retention capacity of the water-absorbent resin tends to decrease. Accordingly, the amount of the post-crosslinking agent used is usually 0.00001 to 0.01 mol, preferably 0.00005 to 0.005 mol, and more preferably 0.0001 to 0.002 mol per mole of the total amount of water-soluble ethylenic unsaturated monomer used for polymerization.

The timing for adding the post-crosslinking agent is not particularly limited as long as it is added after polymerization is completed. The post-crosslinking agent is preferably added in the presence of 1 to 400 parts by mass of water, more preferably 5 to 200 parts by mass of water, and most preferably 10 to 100 parts by mass of water relative to the total 100 parts by mass of the water-soluble ethylenic unsaturated monomer used to obtain the water-absorbent resin.

When the post-crosslinking agent is used, water or a hydrophilic organic solvent may be used as a solvent, if necessary. Examples of hydrophilic organic solvents include lower alcohols, such as methyl alcohol, ethyl alcohol, n-propyl alcohol, and isopropyl alcohol; ketones, such as acetone and methyl ethyl ketone; ethers, such as diethyl ether, dioxane, and tetrahydrofuran; amides, such as N,N-dimethylformamide; sulfoxides, such as dimethyl sulfoxide; and the like. These hydrophilic organic solvents may be used singly, or in a combination of two or more thereof. The hydrophilic organic solvent(s) may also be used as a mixed solvent with water.

The reaction temperature in the post-crosslinking reaction is preferably 50 to 250°C, more preferably 60 to 180°C, yet more preferably 60 to 140°C, and still more preferably 70 to 120°C. Further, although the post-crosslinking reaction time cannot be generally specified because it varies depending on the reaction temperature, type and amount of the crosslinking agent, etc., the post-crosslinking reaction time is typically 1 to 300 minutes and preferably 5 to 200 minutes.

(4) Drying

In the present invention, drying may be carried out under normal pressure or reduced pressure, and may also be carried out under a nitrogen stream or the like in order to increase the drying efficiency. When drying is carried out under normal pressure, the drying temperature is preferably 70 to 250°C, more preferably 80 to 180°C, yet more preferably 80 to 140°C, and still more preferably 90 to 130°C. When the drying is carried out under reduced pressure, the drying temperature is preferably 60 to 100°C and more preferably 70 to 90°C.

Absorbent Material and Absorbent Article

The absorbent material of the present invention comprises the above-described water-absorbent resin of the present invention and hydrophilic fibers. Examples of the structure of the absorbent material include a mixing structure obtained by uniformly blending water-absorbent resin and hydrophilic fibers; a sandwich structure in which the water-absorbent resin is sandwiched between layered hydrophilic fibers; and a structure in which the water-absorbent resin and hydrophilic fibers are wrapped with tissue paper. However, the present invention is not limited to these examples.

Examples of hydrophilic fibers include cellulose fibers, such as cotton-like pulp obtained from wood, mechanical pulp, chemical pulp, and semi-chemical pulp; artificial cellulose fibers, such as rayon and acetate; and fibers formed from synthetic resins, such as hydrophilic-treated polyamide, polyester, and polyolefin. However, the present invention is not limited to these examples.

Further, the absorbent material of the present invention may also contain other components, such as adhesive binders including thermal-fusible synthetic fibers, hot melt adhesives, adhesive emulsions, and the like in order to improve the shape-retaining property of the absorbent material.

The content of the water-absorbent resin in the absorbent material of the present invention is preferably 5 to 80% by mass, more preferably 10 to 70% by mass, and still more preferably 15 to 60% by mass. When the content of the water-absorbent resin is too small, the absorption capacity of the absorbent material tends to decrease, leading to an increase in liquid leakage and re-wetting. Further, when the content of the water-absorbent resin is too large, the cost of the absorbent material tends to increase and the texture of the absorbent material tends to harden.

The above-described absorbent material of the present invention is held between a liquid-permeable sheet (top sheet) that allows liquid to permeate and a liquid-impermeable sheet (back sheet) that does not allow liquid to permeate, and an absorbent article can thereby be formed. The liquid-permeable sheet is positioned on the side contacting the body and the liquid-impermeable sheet is positioned on the side opposite to the side contacting the body.

Examples of liquid-permeable sheets include non-woven fabrics and porous synthetic resin sheets, which are formed from polyethylene, polypropylene, polyesters, polyamide, and the like. Examples of liquid-impermeable sheets include films formed from synthetic resins, such as polyethylene, polypropylene, and polyvinyl chloride; films formed from composite materials of these synthetic resins and non-woven fabrics; and the like. However, the present invention is not limited to these examples.

The type of absorbent article is not particularly limited. Representative examples of absorbent articles include hygienic materials, such as disposable diapers, sanitary napkins, and incontinence pads; urine-absorbing materials for pets; materials for civil engineering and construction, such as packing materials; food freshness preservers, such as drip absorbents and refrigerants; agricultural and horticultural materials, such as water-retaining materials for soil, and the like.

Advantageous Effect of the Invention

The water-absorbent resin of the present invention has properties such as a high water-retention capacity and a high gel strength. Accordingly, the use of the water-absorbent resin of the present invention can provide an absorbent material having excellent properties in terms of liquid permeation rate and the like, as well as various absorbent articles that use the absorbent material. In particular, the absorbent resin of the present invention can be suitably used in hygienic materials such as disposable diapers.

Brief Description of the Drawing

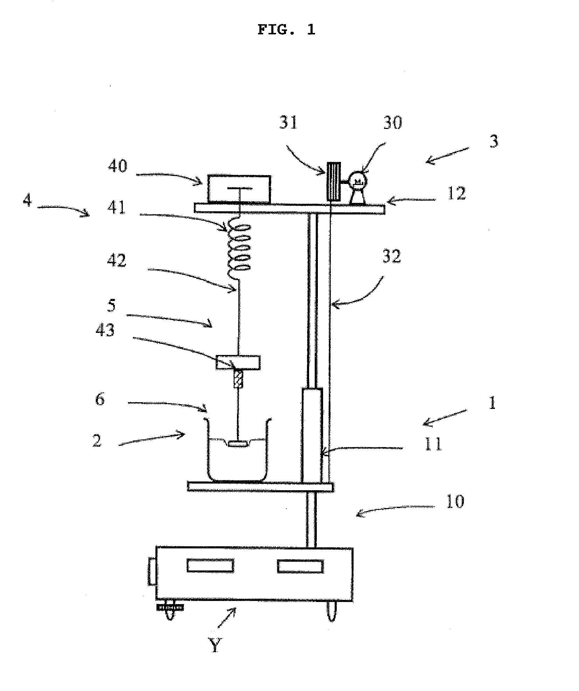

[Fig. 1] A schematic view showing a general structure of a device for measuring the gel strength of the absorbent resin.

Description of Embodiments

The present invention is explained in detail below with reference to Examples and Comparative Examples; however, the present invention is not limited to the Examples below.

The water-absorbent resin obtained in each Example and Comparative Example was evaluated in terms of the water-retention capacity of saline solution, median particle size, and gel strength by the following methods.

<Water-Retention Capacity of Saline Solution>

500 g of a 0.9% by mass aqueous solution of sodium chloride (saline solution) was weighed out in a 500-mL beaker. 2.0 g of water-absorbent resin was dispersed therein so as not to form lumps while stirring the solution at 600 rpm. The solution was left to stand for 30 minutes in a stirred state to sufficiently swell the water-absorbent resin. Subsequently, the solution was poured into a cotton bag (Cottonbroad No. 60, 100 mm (W) x 200 mm (H)), and the upper part of the cotton bag was closed with a rubber band. The cotton bag was dehydrated for 1 minute using a dehydrator (produced by Kokusan Co., Ltd., product number: H-122) that was set at a centrifugal force of 167 G, and the mass Wa (g) of the dehydrated cotton bag containing swollen gel was measured. The same procedure was repeated without adding the water-absorbent resin, and the mass Wb (g) of the dehydrated empty cotton bag was measured. The water-retention capacity was calculated by the following equation:

```
Water-retention capacity of saline solution (g/g) =

     [Wa-Wb](g)/mass of the water-absorbent resin (g)
```

<Median Particle Size>

0.25 g of amorphous silica (Degussa Japan, Sipernat 200) was mixed as a lubricant with 50 g of a water-absorbent resin.

JIS standard sieves having openings of 850 $\mu$m, 600 $\mu$m, 500 $\mu$m, 425 $\mu$m, 300 $\mu$m, 250 $\mu$m, and 150 $\mu$m, and a receiving tray were combined in that order from the top. Water-absorbent resin were placed on the top sieve of the combined sieves, and shaken for 20 minutes using a Ro-Tap Sieve Shaker to conduct classification.

After classification, the mass of the water-absorbent resin remaining on each sieve was calculated as the mass percentage relative to the total mass, and the mass percentage was integrated in descending order of particle size. Thereby, the relationship between the sieve opening and the integrated value of the mass percentage of the water-absorbent resin remaining on the sieve was plotted on a logarithmic probability paper. By connecting the plots on the probability paper with straight lines, the particle size corresponding to the 50% percentile of the integrated mass percentage was obtained as the median particle size.

<Gel Strength of Water-Absorbent Resin>

The gel strength of the water-absorbent resin of the present invention is a value measured using a device Y (such as the Neocardmeter, M-303 model, produced by IioDenki Kogyo)having the measurement principle as shown in Fig. 1, under the following conditions.

The device Y comprises a supporting member 1, a movable base plate 2 for holding the measuring sample (gel) 6, a driving member 3 for driving the movable base plate 2, and a measuring member 4.

In the supporting member 1, a base 12 is fixed to the upper portion of a supporting pole 11 standing on a supporting plate 10. The movable base plate 2 is attached to the supporting pole 11 so as to be vertically movable. On the base 12, a pulse motor 30 is mounted and the movable base plate 2 is moved up and down via a wire 32 by rotating a pulley 31.

In the measuring member 4, a pressure-sensitive shaft 43 with a disc is equipped, via a precision spring 41 and a connection shaft 42, to a load cell 40 for measuring distortion due to deformation. Depending on the measurement conditions, the diameter of the disc can be changed. A weight 5 may be placed on the pressure-sensitive shaft 43 with a disc.

The working principle of the device Y is described below.

The precision spring 41 is fixed to the load cell 40 (stress detector) disposed on the precision spring 41 and connected to the pressure-sensitive shaft 43 with a disc disposed below the precision spring 41. Having a predetermined weight 5 placed thereon, the pressure-sensitive shaft 43 with a disc is vertically suspended. The movable base plate 2 having a measuring sample 6 placed thereon moves upward due to the rotation of the pulse motor 30 at a fixed speed. A constant rate of load is applied to the sample 6 via the precision spring 41, and the distortion due to deformation is measured by the load cell 40 and processed to attain a hardness measurement.

29.0 g (for 30 times swelling) of saline solution was weighed out in a 100-mL beaker, and a magnetic stirrer bar (8 mm (diameter) x 30 mm, without a ring) was placed therein, and the beaker was placed on a magnetic stirrer (produced by Iuchi, model number: HS-30D). The rotation of the magnetic stirrer bar was then adjusted to 600 rpm.

Next, 1.0 g of the water-absorbent resin was added to the beaker while stirring. The stirring was continued until the vortex disappeared and the liquid surface became flat, thereby obtaining a gel (measuring sample 6).

One hour later, the hardness of the gel was measured using device Y (Neocardmeter, M-303 model, produced by IioDenki Kogyo, diameter of the disc of the pressure-sensitive shaft: 16 mm, load: 100 g, carriage speed: 7 seconds/inch, and measurement mode: viscous). Based on the resulting hardness value (dyne/cm$^2$), the gel strength a was calculated using the following equation (0.1: unit correction factor (dyne/cm$^2$→Pa)).

$$\texttt{Gel strength } \mathbf{a} \texttt{ (Pa) = [hardness value]×0.1}$$

Next, gel strength **b** and gel strength **C** were measured using the same procedure except that the amounts of saline solution were changed to 39.0 g (for 40 times swelling) and 49.0 g (for 50 times swelling), respectively.

The index represented by the sum of the gel strengths **a, b,** and **c** is determined to be the total gel strength.

$$\texttt{Total gel strength (Pa) = Gel strength } \mathbf{a} \texttt{ + Gel strength } \mathbf{b} \texttt{ + Gel strength } \mathbf{C}$$

[Example 1]

A 2-L cylindrical round-bottomed separable flask having an internal diameter of 110 mm, equipped with a stirrer, a two pitched paddle impeller, a reflux condenser, a dropping funnel, and a nitrogen gas inlet tube was prepared in a draft chamber.

290 g of n-heptane was placed into the flask, and 0.74 g of a sucrose stearate (produced by Mitsubishi-Kagaku Foods Corporation, Ryoto sugar ester S-370) and 0.74 g of a maleic anhydride-modified ethylene-propylene copolymer (produced by Mitsui Chemicals, Inc., Hi-wax 1105A) were added thereto. The temperature was raised to 80°C while stirring to dissolve the surfactant, and thereafter the solution was cooled to 50°C.

Separately, 92 g (1.02 mol) of an 80% by mass aqueous solution of acrylic acid was placed into a 500-mL Erlenmeyer flask, and 146.0 g of a 21% by mass aqueous solution of sodium hydroxide was added dropwise thereto under external cooling to perform 75 mol% neutralization. Thereafter, 0.030 g (0.111 mmol) of 2,2'-azobis(2-amidinopropane)dihydrochloride as a radical polymerization initiator, 0.010 g (0.0649 mmol) of N,N'-methylenebisacrylamide as an internal-crosslinking agent, and 5.4 mg (0.0509 mmol) of sodium hypophosphite monohydrate as a chain transfer agent were added thereto and dissolved, thereby preparing an aqueous monomer solution for the first step.

The entire amount of aqueous monomer solution for the first step was added to the separable flask, and the interior of the system was sufficiently replaced with nitrogen. Thereafter, the flask was immersed in a water bath at 70°C to raise the temperature, and the first-step polymerization was performed for 30 minutes, thereby obtaining the reaction mixture of the first step.

Separately, 128.8 g (1.43 mol) of an 80% by mass aqueous solution of acrylic acid was placed into another 500-mL Erlenmeyer flask, and 159.0 g of a 27% by mass aqueous solution of sodium hydroxide was added dropwise thereto under external cooling to perform 75 mol% neutralization. Thereafter, 0.042 g (0.155 mmol) of 2,2'-azobis(2-amidinopropane)dihydrochloride as a radical polymerization initiator, 0.012 g (0.0778 mmol) of N,N'-methylenebisacrylamide as an internal-crosslinking agent, and 7.6 mg (0.0717 mmol) of sodium hypophosphite monohydrate as a hypophosphorous acid compound were added thereto and dissolved, thereby preparing an aqueous monomer solution for the second step.

The reaction mixture of the first step was cooled to 28°C. The aqueous monomer solution for the second step was added thereto at the same temperature, and allowed to be absorbed for 30 minutes. At the same time, the interior of the system was sufficiently replaced with nitrogen. Thereafter, the flask was again immersed in a water bath at 70°C to raise the temperature, and the second-step polymerization was performed for 30 minutes.

After the second-step polymerization, the reaction mixture was heated using an oil bath at 125°C, and 240 g of water was distilled off to the outside of the system while refluxing n-heptane using azeotropic distillation of water and n-heptane. Thereafter, 4.42 g (0.51 mmol) of a 2% by mass aqueous solution of ethylene glycol diglycidyl ether was added thereto, and the mixture was subjected to a post-crosslinking reaction at 80°C for 2 hours. Subsequently, the reaction mixture was heated using an oil bath at 125°C and dried by evaporating n-heptane, thereby obtaining 230.3 g of water-absorbent resin. Table 1 shows the measurement results of each property.

[Example 2]

The same procedure as in Example 1 was repeated except that the amount of the water distilled off to the outside of the system after conduction of the second-step polymerization was changed to 245 g, thereby obtaining 228.7 g of water-absorbent resin. Table 1 shows the measurement results of each property.

[Example 3]

The same procedure as in Example 1 was repeated except that the amount of the water distilled off to the outside of the system after conduction of the second-step polymerization was changed to 250 g, thereby obtaining 226.9 g of water-absorbent resin. Table 1 shows the measurement results of each property.

[Example 4]

The same procedure as in Example 2 was repeated except that the amount of 2,2'-azobis(2-amidinopropane) dihydrochloride as a radical polymerization initiator used in the first step was changed to 0.018 g (0.0664 mmol), and that used in the second step was changed to 0.025 g (0.0922 mmol), and the amount of sodium hypophosphite monohydrate as a chain transfer agent used in the first step was changed to 3.8 mg (0.0359 mmol), and that used in the second step was changed to 5.3 mg (0.0500 mmol), thereby obtaining 230.7 g of water-absorbent resin. Table 1 shows the measurement results of each property.

[Example 5]

The same procedure as in Example 2 was repeated except that the amount of 2,2'-azobis(2-amidinopropane) dihydrochloride as a radical polymerization initiator used in the first step was changed to 0.051 g (0.188 mmol), and that used in the second step was changed to 0.072 g (0.265 mmol), thereby obtaining 229.1 g of water-absorbent resin. Table 1 shows the measurement results of each property.

[Example 6]

The same procedure as in Example 2 was repeated except that the amount of 2,2'-azobis(2-amidinopropane) dihydrochloride as a radical polymerization initiator used in the first step was changed to 0.044 g (0.162 mmol), and that used in the second step was changed to 0.061 g (0.225 mmol), and the amount of sodium hypophosphite monohydrate as a chain transfer agent used in the first step was changed to 0.015 g (0.142 mmol), and that used in the second step was changed to 0.021 g (0.198 mmol), thereby obtaining 228.2 g of water-absorbent resin. Table 1 shows the measurement results of each property.

[Example 7]

The same procedure as in Example 1 was repeated except that the amount of 2,2'-azobis(2-amidinopropane) dihydrochloride as a radical polymerization initiator used in the first step was changed to 0.044 g (0.162 mmol), and that used in the second step was changed to 0.061 g (0.225 mmol), and the amount of sodium hypophosphite monohydrate as a chain transfer agent used in the first step was changed to 0.020 g (0.189 mmol), and that used in the second step was changed to 0.028 g (0.264 mmol), thereby obtaining 230.2 g of water-absorbent resin. Table 1 shows the measurement results of each property.

[Comparative Example 1]

A 2-L cylindrical round-bottomed separable flask having an internal diameter of 110 mm, equipped with a stirrer, a two pitched paddle impeller, a reflux condenser, a dropping funnel, and a nitrogen gas inlet tube was prepared in a draft chamber.

290 g of n-heptane was placed into the flask, and 0.74 g of a sucrose stearate (produced by Mitsubishi-Kagaku Foods Corporation, Ryoto sugar ester S-370) and 0.74 g of a maleic anhydride-modified ethylene-propylene copolymer (produced by Mitsui Chemicals, Inc., Hi-wax 1105A) were added thereto. The temperature was raised to 80°C while stirring to dissolve the surfactant, and thereafter the solution was cooled to 50°C.

Separately, 92 g (1.02 mol) of an 80% by mass aqueous solution of acrylic acid was placed into a 500-mL Erlenmeyer flask, and 146.0 g of a 21% by mass aqueous solution of sodium hydroxide was added dropwise thereto under external cooling to perform 75 mol% neutralization. Thereafter, 0.11 g (0.41 mmol) of potassium persulfate as a radical polymerization initiator, and 0.010 g (0.0649 mmol) of N,N'-methylenebisacrylamide as an internal-crosslinking agent were added thereto and dissolved, thereby preparing an aqueous monomer solution for the first step.

The entire amount of aqueous monomer solution for the first step was added to the separable flask, and the interior of the system was sufficiently replaced with nitrogen. Thereafter, the flask was immersed in a water bath at 70°C to raise the temperature, and the first-step polymerization was performed for 30 minutes, thereby obtaining the reaction mixture of the first step.

Separately, 128.8 g (1.43 mol) of an 80% by mass aqueous solution of acrylic acid was placed into another 500-mL Erlenmeyer flask, and 159.0 g of a 27% by mass aqueous solution of sodium hydroxide was added dropwise thereto under external cooling to perform 75 mol% neutralization. Thereafter, 0.039 g (0.144 mmol) of potassium persulfate as a radical polymerization initiator, and 0.012 g (0.0778 mmol) of N,N'-methylenebisacrylamide as an internal-crosslinking agent were added thereto and dissolved, thereby preparing an aqueous monomer solution for the second step.

The reaction mixture of the first step was cooled to 28°C. The aqueous monomer solution for the second step was added thereto at the same temperature, and allowed to be absorbed for 30 minutes. At the same time, the interior of the system was sufficiently replaced with nitrogen. Thereafter, the flask was again immersed in a water bath at 70°C to raise the temperature, and the second-step polymerization was performed for 30 minutes.

After the second-step polymerization, the reaction mixture was heated using an oil bath at 125°C, 240 g of water was distilled off to the outside of the system while refluxing n-heptane using azeotropic distillation of water and n-heptane. Thereafter, 4.42 g (0.51 mmol) of a 2% by mass aqueous solution of ethylene glycol diglycidyl ether was added thereto, and the mixture was subjected to a post-crosslinking reaction at 80°C for 2 hours. Subsequently, the reaction mixture was heated using an oil bath at 125°C and dried by evaporating n-heptane, thereby obtaining 229.3 g of water-absorbent resin. Table 1 shows the measurement results of each property.

[Comparative Example 2]

The same procedure as in Comparative Example 1 was repeated except that the amount of the water distilled off to the outside of the system after conducting the second-step polymerization was changed to 250 g, thereby obtaining 227.7 g of water-absorbent resin. Table 1 shows the measurement results of each property.

[Comparative Example 3]

The same procedure as in Comparative Example 1 was repeated except that the amount of the water distilled off to the outside of the system after conducting the second-step polymerization was changed to 258 g, thereby obtaining 227.5 g of water-absorbent resin. Table 1 shows the measurement results of each property.

[Comparative Example 4]

The same procedure as in Example 2 was repeated except that the amount of 2,2'-azobis(2-amidinopropane) dihydrochloride as a radical polymerization initiator used in the first step was changed to 0.012 g (0.0442 mmol), and that used in the second step was changed to 0.017 g (0.0627 mmol), thereby obtaining 229.5 g of water-absorbent resin. Table 1 shows the measurement results of each property.

[Comparative Example 5]

The same procedure as in Example 3 was repeated except that the amount of sodium hypophosphite monohydrate as a chain transfer agent used in the first step was changed to 1.0 mg (0.00943 mmol), and that used in the second step was changed to 1.4 mg (0.0132 mmol), thereby obtaining 225.2 g of water-absorbent resin. Table 1 shows the measurement results of each property.

[Comparative Example 6]

The same procedure as in Example 3 was repeated except that the amount of 2,2'-azobis(2-amidinopropane) dihydrochloride as a radical polymerization initiator used in the first step was changed to 0.060 g (0.221 mmol), and that used in the second step was changed to 0.084 g (0.310 mmol), and sodium hypophosphite monohydrate as a chain transfer agent was not used, thereby obtaining 226.4 g of water-absorbent resin. Table 1 shows the measurement results of each property.

Using the water-absorbent resin obtained in each Example and Comparative Example, an absorbent material and an absorbent article were produced and evaluated following the methods described below. Table 1 shows the results.

<Performance of Absorbent Article>

(a) Preparation of Artificial Urine

Distilled water (q.s.), sodium chloride (60 g), calcium chloride dihydrate (1.8 g), and magnesium chloride hexahydrate (3.6 g) were placed into a 10-L container, and dissolved. Subsequently, 0.02 g of poly(oxyethylene)nonylphenyleter was added thereto, and distilled water was further added to adjust the total mass of the aqueous solution to 6,000 g. The solution was colored with a small amount of Blue No. 1, thereby preparing artificial urine.

(b) Production of Absorbent Article

10 g of water-absorbent resin and 10 g of crushed pulp (product name: Rayfloc, produced by Rayoneir) were uniformly mixed by an air sheet making technique to form a sheet-like absorbent material core having a size of 42 cm x 12 cm. The absorbent material core was then pressed by a roll press while being sandwiched between two pieces of tissue paper each having the same size as the absorbent material core and basis weight of 16 g/m$^2$ to obtain an absorbent material. Thereafter, an air-through-type porous liquid-permeable sheet made of polyethylene having the same size as the absorbent material and a basis weight of 22 g/m$^2$ was placed over the absorbent material, and a liquid-impermeable sheet made of polyethylene having the same size and weight was placed below the absorbent material to sandwich the absorbent material, thereby obtaining an absorbent article.

(c) Liquid Permeation Time

In order to evaluate the liquid permeation rate, the liquid permeation time was measured in the following manner. The shorter the liquid permeation time, the faster the liquid permeation rate would be.

An absorbent article was placed on a horizontal base. A measurement device was placed in the central portion of the absorbent article. The measurement device was provided with a liquid-injection cylinder having an inside diameter of 3 cm that was positioned in the center of a weight having a size of 10 cm x 10 cm and a weight of 2 kg. 50 mL of artificial urine was poured into the cylinder at once and a stopwatch was used to measure the time required for the artificial urine to completely disappear from the cylinder. This was regarded as the first liquid permeation time (seconds). Next, the cylinder was removed, and the absorbent article was stored as is. At 30 minutes and 60 minutes, respectively, after the start of the first injection of the artificial urine, the measurement device was placed at the same position as the first time, and the same procedure was carried out to measure the liquid permeation time (seconds) for the second and third times. The total time in seconds of the first to third procedures was determined to be the total liquid permeation time. The shorter the liquid permeation time, the more desirable the absorbent article would be. For example, the total liquid permeation time is preferably 300 seconds or less.

(d) Amount of Re-wet

After 60 minutes had elapsed from the completion of measuring the liquid permeation time, sheets of filter paper of 10 cm each side whose mass had been measured in advance ((Wc (g), approximately 50 g) were placed near the position for pouring the artificial urine on the absorbent article, and a 5-kg weight having a bottom area of 10 cm × 10 cm was placed on the filter paper. After the load was applied for 5 minutes, the mass of filter paper (Wd (g)) was measured, and the increased mass was regarded as the amount of re-wet (g). The smaller the amount of re-wet, the more preferable the absorbent article would be. For example, the amount of re-wet is preferably 8 g or less.

$$\texttt{Amount of re-wet (g) = Wd-Wc}$$

Table 1

| | Median particl e size | Water-retention capacity of saline solution | Gel strength [Pa] | | | | Performance of absorbent article | |
|---|---|---|---|---|---|---|---|---|
| | | | | | | | Amount of re-wet | Total liquid permeation time |
| | [$\mu$m] | [g/g] | a | b | c | Total gel strengt h | [g] | [sec] |
| Example 1 | 430 | 41 | 3800 | 2200 | 1590 | 7590 | 6.3 | 173 |
| Example 2 | 420 | 45 | 3200 | 2000 | 1350 | 6550 | 2.0 | 256 |
| Example 3 | 430 | 51 | 3300 | 1970 | 1480 | 6750 | 1.0 | 281 |
| Example 4 | 420 | 43 | 3320 | 2010 | 1360 | 6690 | 3.7 | 253 |
| Example 5 | 410 | 44 | 3510 | 2060 | 1400 | 6970 | 2.5 | 232 |
| Example 6 | 420 | 48 | 3240 | 1910 | 1430 | 6580 | 1.3 | 276 |
| Example 7 | 440 | 49 | 3100 | 1870 | 1400 | 6370 | 1.5 | 292 |
| Comparative example 1 | 410 | 30 | 2800 | 2060 | 1240 | 6100 | 19.1 | 209 |
| Comparative example 2 | 420 | 40 | 1900 | 1400 | 980 | 4280 | 15.6 | 337 |
| Comparative example 3 | 410 | 51 | 1970 | 1350 | 1160 | 4480 | 5.9 | 358 |
| Comparative example 4 | 420 | 46 | 2480 | 1420 | 1000 | 4900 | 10.7 | 324 |
| Comparative example 5 | 440 | 48 | 2500 | 1590 | 1190 | 5280 | 5.3 | 331 |
| Comparative example 6 | 430 | 45 | 2520 | 1450 | 1120 | 5090 | 9.1 | 315 |

As is clear from Table 1, the water-absorbent resins obtained in Examples 1 to 7 had a high total gel strength in the region with high water-retention capacity of saline solution. Table 1 also indicates that the absorbent article obtained in each Example exhibited excellent performance both in amount of re-wet and total liquid permeation time.

In contrast, the water-absorbent resin obtained in Comparative Example 1 had a relatively high total gel strength; however, due to its low water-retention capacity of saline solution, the resulting absorbent article exhibited a large amount of re-wet. Furthermore, the water-absorbent resins obtained in Comparative Examples 2 to 6 had a relatively high water-retention capacity of saline solution, but due to their low total gel strength, gel blocking occurred, resulting in a slow liquid permeation rate for the resulting absorbent articles.

[Explanation of numerical symbols]

Y       Gel strength measuring device
1       Supporting member
10      Supporting plate
11      Supporting pole
12      Base
2       Movable base plate
3       Movable base plate driving member
30      Pulse motor
31      Pulley
32      Wire
4       Measuring member
40      Load cell
41      Precision spring
42      Connection shaft
43      Pressure-sensitive shaft
5       Weight
6       measuring sample (Gel)

**Claims**

1.  A water-absorbent resin satisfying criteria (1) and (2) below:

    (1) having a water-retention capacity of saline solution of 40 g/g or more, and
    (2) having a total gel strength of 5,500 Pa or greater, the total gel strength being the sum of 30 times swollen gel strength **a,** 40 times swollen gel strength **b,** and 50 times swollen gel strength **c,**

    the 30 times swollen gel strength **a** being a gel strength when swelling being performed by adding saline solution to a gel in an amount with the total mass of the gel and saline solution being 30 times that of the gel,
    the 40 times swollen gel strength b being a gel strength when swelling being performed by adding saline solution to a gel in an amount with the total mass of the gel and saline solution being 40 times that of the gel, and
    the 50 times swollen gel strength c being a gel strength when swelling being performed by adding saline solution to a gel in an amount with the total mass of the gel and saline solution being 50 times that of the gel.

2.  An absorbent material comprising the water-absorbent resin of claim 1 and hydrophilic fiber.

3.  An absorbent article comprising the absorbent material of claim 2 held between a liquid-permeable sheet and a liquid-impermeable sheet.

FIG. 1

| INTERNATIONAL SEARCH REPORT | International application No. |
|---|---|
| | PCT/JP2012/060627 |

A. CLASSIFICATION OF SUBJECT MATTER
*C08F20/06*(2006.01)i, *A61F13/49*(2006.01)i, *A61F13/53*(2006.01)i, *B01J20/26*(2006.01)i, *B32B27/00*(2006.01)i

According to International Patent Classification (IPC) or to both national classification and IPC

B. FIELDS SEARCHED

Minimum documentation searched (classification system followed by classification symbols)
C08F20/06, A61F13/49, A61F13/53, B01J20/26, B32B27/00, C08F2/00-2/60

Documentation searched other than minimum documentation to the extent that such documents are included in the fields searched
Jitsuyo Shinan Koho      1922-1996    Jitsuyo Shinan Toroku Koho    1996-2012
Kokai Jitsuyo Shinan Koho   1971-2012    Toroku Jitsuyo Shinan Koho    1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2006/123561 A1 (Sumitomo Seika Chemicals Co., Ltd.), 23 November 2006 (23.11.2006), claims; paragraphs [0037], [0038]; examples & US 2008/0280154 A1    & EP 1882701 A1 | 1-3 |
| A | JP 2005-111474 A (Nippon Shokubai Co., Ltd.), 28 April 2005 (28.04.2005), claims; examples & JP 2011-218357 A      & US 2007/0123658 A1 & US 2010/0308263 A1    & EP 1677845 A1 & WO 2005/027986 A1    & KR 10-2006-0072148 A & CN 1856331 A | 1-3 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 May, 2012 (16.05.12) | 29 May, 2012 (29.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

<table>
<tr><td colspan="2" align="center">**INTERNATIONAL SEARCH REPORT**</td><td>International application No.<br>PCT/JP2012/060627</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| P,A | WO 2012/023433 A1  (Sumitomo Seika Chemicals Co., Ltd.),<br>23 February 2012 (23.02.2012),<br>claims; paragraph [0025]; examples<br>(Family: none) | 1-3 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H6345819 B
- JP H3227301 B
- JP H8120013 B
- JP H6287233 B
- JP H9124710 B